# EUROPEAN PATENT SPECIFICATION

(11) **EP 4 327 778 B1**
(45) Date of publication and mention of the grant of the patent: **22.04.2026**
(21) Application number: 23193514.9
(22) Date of filing: 25.08.2023
(51) Int. Cl.: A61F 2/16

(54) **ACCOMMODATING INTRAOCULAR LENS COMPRISING A COMBINATION OF MULTIPLE VARIABLE LENSES**
AKKOMMODATIVE INTRAOKULARLINSE MIT EINER KOMBINATION AUS MEHREREN VARIABLEN LINSEN
LENTILLE INTRAOCULAIRE D'ACCOMMODATION COMPRENANT UNE COMBINAISON DE MULTIPLES LENTILLES VARIABLES

(30) Priority: 26.08.2022 NL 2032859
(43) Date of publication of application: 28.02.2024
(73) Proprietor: Akkolens International B.V., 4836 BA Breda (NL)
(72) Inventor: ROMBACH, Michiel Christiaan, 4836 BA Breda (NL); VAN LAWICK, Willem Pieter, 4836 BA Breda (NL)
(74) Representative: Patentwerk B.V.

(56) References cited:
- WO-A1-2011/053143
- WO-A1-2012/105843
- WO-A1-2020/231260

## Description

The present invention relates to an accommodating intraocular lens construction as defined in claim 1. The accommodating intraocular lens construction comprises a combination of variable lenses (optical elements) including, firstly, a variable lens comprising two spherical lenses fitted onto two optical elements to provide variable optical power of which the degree depends on the degree of movement of at least one of spherical lens along the optical axis and, secondly, a variable lens comprising at least two cubic surfaces fitted onto the same two optical elements which provides a lens of variable optical power of which the degree depends on the degree of movement of at least one of the optical elements in a direction perpendicular to the optical axis. The combination of variable lenses is fitted into a mechanical construction providing said movements of the optical elements.

Accommodating intraocular lenses provide the eye with adjustment of focus power. Such lenses can comprise multiple optical elements each fitted with at least one cubic optical shape or partially cubic function shaped or free-formed. The combination of, for example, two, such cubic optical shapes provide a lens of variable optical power with the degree of optical power depends on the degree of movement, in this example shift, of the optical elements in, opposite, lateral directions, meaning: directions perpendicular to the optical axis, henceforth abbreviated as the 'lateral variable lens'. Such variable lenses are disclosed in, for example, US2008046076 and WO2005084587 and related documents.

Such lenses can, alternatively, also comprise multiple optical elements each fitted with at least one largely spherical optical shape. The combination of, for example, two, such spherical optical shapes provide a lens of variable optical power with the degree of optical power depending on the degree of movement, in this example axial travel, of the optical elements in, generally opposite but not necessarily so, axial direction, meaning: directions along the optical axis, henceforth abbreviated as the 'axial variable lens'. Such variable lenses are disclosed in, for example, WO2011062486 and US20020002404 and related documents.

WO2011053143 discloses an intraocular lens comprising at least one lens for variable focus comprising at least two optical elements with each of these elements comprising at least one surface or optical function which surfaces include: one surface for focusing by lateral movement provided on one optical element which surface is adapted to provide variable focus of which the degree of focus depends on the lateral position of the optical element, one surface for focusing by axial movement provided on any other optical element which surface is adapted to provide variable focus of which the degree of focus depends on the axial position of the optical element, wherein the lens comprises at least one connecting component for simultaneous movement connected to the optical elements adapted to provide simultaneous mutual movements of the optical elements along at least one lateral axis and along the axial axis and that the variable focus of the lens is a combination of variable focus provided by the surface for focusing by lateral movement and variable focus provided by the surface for focusing by axial movement.

It is a first object of the present invention to provide a lens construction that can provide improved variable optical power.

The present invention provides thereto an accommodating intraocular lens construction comprising an optical axis with the lens comprising at least two optical elements with each element comprising two optical surfaces,
wherein the optical elements are mutually elastically coupled by at least one elastic haptic, and
wherein at least one, preferably each, optical element comprises at least two optical subelements wherein at least one optical subelement comprises an optical surface that is at least partially cubic function shaped or free-formed and wherein at least one other optical subelement comprises at least one optical surface that is at least partially spherical shaped, and
wherein the elastic haptic is configured to provide, at compression of the lens construction in a lateral direction, said lateral direction being directed perpendicular to the optical axis, a combination of lateral movements in lateral direction and axial movements in axial direction of the optical elements of the optical elements,
wherein the axial movements are directed along the optical axis.

The present invention discloses an intraocular lens construction. The lens construction is advantageous because it can provide variable optical power due to the combination of at least one lateral variable lens (optical element) and at least one axial variable lens (optical element) and an elastic haptic. The lens construction according to the present invention provides said, independent, movements of the optical elements. The lens construction according to the present invention can provide a combination of lateral and axial movements of the optical elements and as such provide a variable optical power.

Each optical element according to the present invention comprises at least two, preferably two, optical surfaces. One of these surfaces is at least partially spherical shaped. Preferably, this surface is the outer surface, facing outwards the lens construction. Another optical surface of the same optical element is at least partially formed according to the shape of a graph resulting from a cubic formula, which can be referred to as cubic optical shape in this document and/or as partially cubic function shaped and/or free-formed. The partially cubic function shaped or free-formed is preferably positioned as the inner surface in the optical element, facing inwards the lens construction. The at least partially cubic function shaped or free-formed surface is preferably part of an optical subelement that has at least partially the shape of an area under a graph of a cubic formula.

The at least partially spherical shaped surface can form part of another optical subelement that has at least partially the shape of an area under a graph of a spherical formula.

In the present document and illustrations embodiments of the present invention are described. In a preferred embodiment of the present invention the lens construction comprises two independent hinges to allow for the two independent directions of movement of the optical elements.

In an embodiment, the present invention relates to an accommodating intraocular lens construction having an optical axis with the lens comprising at least two optical elements with each element comprising two optical surfaces with the optical elements mutually elastically coupled by at least one elastic haptic with each optical element comprising two optical surfaces with element fitted with at least one at least partially (largely) partially cubic function shaped or free-formed and at least one at least partially (largely) spherical optical shape wherein the elastic haptic is adapted to provide, at compression of the lens construction in a lateral direction, directed perpendicular to the optical axis, a combination of lateral movements of the optical elements and axial movements of the optical elements in a axial direction, directed along the optical axis with the lens construction.

The lens construction according to the present invention may comprise two haptics to fit the lens construction according to the present invention at the sulcus plane of the eye.

Typically, the lens construction according to the present invention comprises a combination of at least two at least partially (largely) cubic function shaped or free-formed surfaces, wherein preferably said surfaces areconfigured such that a lens of variable optical power is provided of which the degree of optical power depends on the degree of mutually opposite movements of the optical elements in a lateral direction.

Optionally, the lens construction may comprise a combination of at least two at least partially (largely) spherical optically shaped surfaces that are configured to provide a lens of variable optical power of which the degree of optical power can depend on the degree of movements of the elements in an axial direction.

In an embodiment, the spherically optically shaped surfaces may be configured such that a lens of variable power is provided of which the degree of optical power depends on the degree of movements of the elements in axial direction, wherein the axial movements of the optical elements can be movements in opposite directions along the optical axis or alternatively, wherein the axial movements of the optical elements are in the same direction along the optical axis.

In another embodiment, the lens construction may comprise a combination of at least two substantially spherical optically shaped surfaces is configured to provide a lens of variable optical power of which the degree of optical power depends on the degree of movements of the elements in an axial direction wherein the axial movements of the optical elements are in the same direction along the optical axis.

In another embodiment, both optical elements can comprise a spherical optical shape of a positive optical power. Preferably, a the lens construction according to the present invention is configured to move both elements in the same direction along the optical axis. Optionally at least one other optical element comprises a spherical optical shape of a negative optical power and preferably the lens construction can be (mechanically) configured to move both elements in opposite directions along the optical axis.

In an embodiment, at least two additional free-form optical surfaces can also be fitted onto the two at least partially (largely) partially cubic function shaped or free-formed surfaces. These free-form optical surfaces can provide variable optical power of at least one additional aberration of which the degree of optical power depends on the degree of mutually opposite movements of the elements in a lateral direction. The lateral direction meant is a direction perpendicular to the optical axis. For example the additional free-form surfaces can provide variable correction of, undesired, variable astigmatism and/or coma due to lateral shift of spherical lenses included in the lens construction, or example, lateral shift versus each other and/or lateral shift versus the cornea of the eye.

In an embodiment of the lens construction, one optical element can comprise a spherical optical shape of a positive optical power with at least one other element comprising a spherical optical shape of a negative optical power with mechanical construction configured to move at least the two (both) elements in opposite directions along the optical axis with the optical elements fitted into the (mechanical) lens construction adapted to provide a combination of lateral and axial movements of the optical elements. According to the invention, the lens construction comprises a combination of at least two, preferably two, sets of hinges comprising at least one (set of) hinge(s) adapted to provide lateral movement of the optical elements and at least one (set of) hinge(s) adapted to provide axial movement of the optical elements. Preferably an embodiment of the (mechanical) lens construction, if hinges are applied, comprise two independent sets of hinges.

In an embodiment of the lens construction according to the present invention, at least one optical surface is shaped like a cubic chape function. Preferably at least one optical surface may be shaped as a Hermitian shape function. At least one optical surface may be shaped as the area under the graph of a Hermitian shape function.

Further advantages, features and details of the invention are elucidated on the basis of preferred embodiments thereof, wherein reference is made to the accompanying drawings, in which:
- Figure 1 schematically shows a perspective view of a lens construction according to the present invention;
- Figure 2 schematically shows a cross section of a lens construction and the effect thereof according to the present invention.

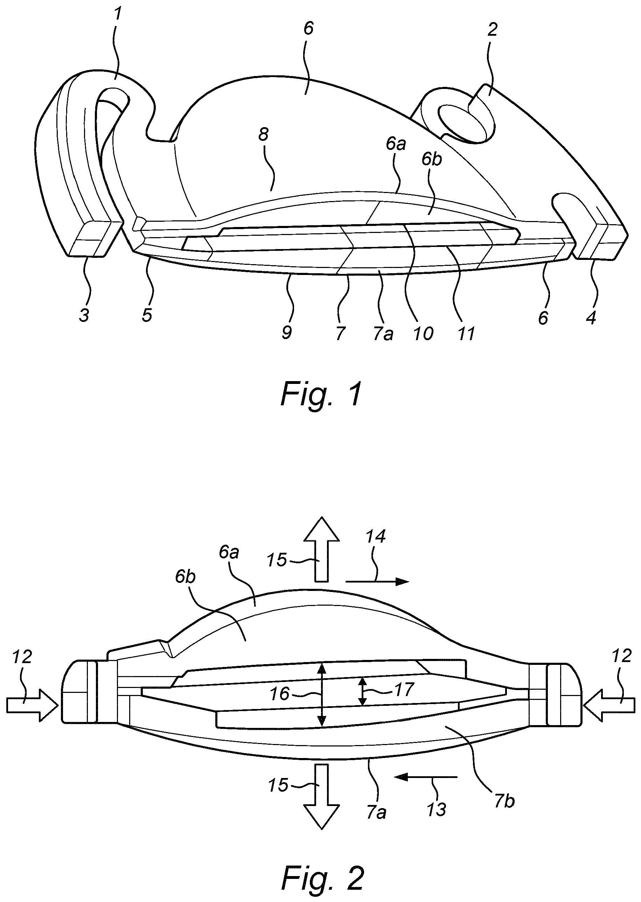

Figure 1 shows an example of an accommodating intraocular lens construction as disclosed in the present document with the lens comprising, in this example, a (mechanical) construction including two haptics, 1,2, to fit the lens at the sulcus plane of the eye, the plane in front of the capsular bag and behind the iris of the eye. The (mechanical) lens construction shown includes a first set of elastical hinges, 3,4, to provide lateral movement of the optical elements and a set of hinges, 5,6, to provide axial movement of the optical elements. The optical elements, 6,7, comprise two spherical optical surfaces, 8,9, and two cubic shaped optical surfaces, 10, 11, forming the intra-lenticular space 16. The two optical surfaces 10,11 shown are cubic function or free-from shaped and have as such the shape of the area under the graph a cubic function. In the presentation shown, the upper optical element 6 comprises one subelement 6a comprising a spherical optical surface 8 and the same optical element comprises another subelement 6b comprising a cubic shaped surface 10. In the presentation shown, the lower optical element 7 comprises one subelement 7a comprising a spherical optical surface 9 and the same optical element comprises another subelement 7b comprising a cubic shaped surface 11.

Figure 2 shows the effect of lateral compression of the lens construction, 12, deforming the lens construction. The optical elements shift in lateral and opposite directions, 13, 14, and both the optical elements move in the axial direction, 15, which mutual displacement increases the intra-lenticular space 16,17 between the optical elements 6,7. The size of the space after deforming is shown with reference number 16, which is an increased size with respect to the also shown size of the space 17 that shows the size of the intra-lenticular space in case the lens construction is at rest.

## Claims

1. An accommodating intraocular lens construction comprising a lens and an optical axis, wherein the lens comprises at least two optical elements with each element comprising two optical surfaces,
wherein the optical elements are mutually elastically coupled by at least one elastic haptic, and
wherein at least one, preferably each, optical element comprises at least two optical subelements wherein at least one optical subelement comprises an optical surface that is at least partially cubic function shaped or free-formed and wherein at least one other optical subelement comprises at least one optical surface that is at least partially spherical shaped, and
wherein the elastic haptic is configured to provide, at compression of the lens construction in a lateral direction, said lateral direction being directed perpendicular to the optical axis, a combination of lateral movements in lateral direction and axial movements in axial direction of the optical elements of the optical elements,
wherein the axial movements are directed along the optical axis,
and wherein the lens construction comprises a combination of at least two independent sets of hinges, in particular two independent sets of hinges, comprising at least one hinge adapted to provide independent lateral movement of the optical elements and at least one hinge adapted to provide independent axial movement of the optical elements.

2. The accommodating intraocular lens construction according to claim 1, comprising two optical elements, wherein the two optical elements are positioned on opposite sides of the lens construction and are divided by an intra-lenticular space between the optical elements.

3. The accommodating intraocular lens construction according to claim 2, wherein the optical elements are configured to shift in lateral and opposite directions such that and both the optical elements move in the axial direction which mutual displacement increases the intra-lenticular space between the optical elements.

4. The accommodating intraocular lens construction according to any of the foregoing claims wherein a combination of at least two at least partially cubic function shaped or free-formed shaped surfaces is configured to provide a lens of variable optical power of which the degree of optical power depends on the degree of mutually opposite movements of the optical elements in a lateral direction.

5. The accommodating intraocular lens construction according to any of the foregoing claims 1-4 wherein a combination of at least two at least partially spherical optically shaped surfaces is configured to provide a lens of variable optical power of which the degree of optical power depends on the degree of movements of the elements in an axial direction.

6. The accommodating intraocular lens construction according to claim 5 wherein the axial movements of the optical elements are movements in opposite directions along the optical axis.

7. The accommodating intraocular lens construction according to claim 5 wherein the axial movements of the optical elements are in the same direction along the optical axis.

8. The accommodating intraocular lens construction according to any of the foregoing claims wherein the at least two optical elements comprise a spherical optical shape of a positive optical power and wherein the lens construction provided with a mechanical construction configured to move at least two optical elements in the same direction along the optical axis.

9. The accommodating intraocular lens construction according to any of the foregoing claims wherein at least one optical element comprises a spherical optical shape of a positive optical power and wherein at least one other optical element comprises a spherical optical shape of a negative optical power, wherein the lens construction is adapted to move both elements in opposite directions along the optical axis.

10. The accommodating intraocular lens construction according to any of the foregoing claims wherein the optical elements also comprise at least two additional free-form optical surfaces configured to provide a variable optical power of at least one additional aberration of which the degree of optical power depends on the degree of mutually opposite movements of the elements in a lateral direction.

11. The accommodating intraocular lens construction according to claim 10 wherein the additional free-form surfaces are configured to provide variable correction of variable astigmatism.

12. The accommodating intraocular lens construction according to claim 10 or 11 wherein the additional free-form surfaces are configured to provide variable correction of variable coma.

13. The accommodating intraocular lens construction according to any of the foregoing claims, wherein the optical subelements within one optical element are in abutting contact (in the lens construction) and are configured for providing a combination of lateral and axial movements of the optical elements and/or of the optical subelements.

14. The accommodating intraocular lens construction according to any of the foregoing claims, wherein the at least two independent sets of hinges comprise one set of hinges adapted to provide lateral movement of the optical elements and one set of hinges adapted to provide axial movement of the optical elements.

15. The accommodating intraocular lens construction according to any of the foregoing claims, wherein at least one optical surface is shaped like a cubic chape function, in particular like a Hermitian shape function.

## Patentansprüche

1. Akkommodierende Intraokularlinsenkonstruktion, umfassend eine Linse und eine optische Achse, wobei die Linse mindestens zwei optische Elemente umfasst,
wobei jedes Element zwei optische Oberflächen umfasst, wobei die optischen Elemente durch mindestens eine elastische Haptik elastisch miteinander gekoppelt sind,
und wobei mindestens ein, bevorzugt jedes optische Element mindestens zwei optische Unterelemente umfasst, wobei mindestens ein optisches Unterelement eine optische Oberfläche umfasst, die mindestens teilweise kubisch ausgebildet oder frei geformt ist, und wobei mindestens ein weiteres optisches Unterelement mindestens eine optische Oberfläche umfasst, die mindestens teilweise sphärisch ausgebildet ist, und
wobei die elastische Haptik dazu konfiguriert ist, bei einer Kompression der Linsenkonstruktion in einer seitlichen Richtung, wobei die seitliche Richtung senkrecht zu der optischen Achse ausgerichtet ist, eine Kombination aus seitlichen Bewegungen in seitlicher Richtung und axialen Bewegungen in axialer Richtung der optischen Elemente der optischen Elemente bereitzustellen,
wobei die axialen Bewegungen entlang der optischen Achse gerichtet sind, und wobei die Linsenkonstruktion eine Kombination aus mindestens zwei unabhängigen Scharniersätzen umfasst, insbesondere zwei unabhängige Scharniersätze, die mindestens ein Scharnier umfassen, das angepasst ist, um eine unabhängige seitliche Bewegung der optischen Elemente bereitzustellen, und mindestens ein Scharnier, das angepasst ist, um eine unabhängige axiale Bewegung der optischen Elemente bereitzustellen.

2. Akkommodierende Intraokularlinsenkonstruktion nach Anspruch 1, umfassend zwei optische Elemente, wobei die zwei optischen Elemente auf gegenüberliegenden Seiten der Linsenkonstruktion positioniert sind und durch einen Intralinsenraum zwischen den optischen Elementen getrennt sind.

3. Akkommodierende Intraokularlinsenkonstruktion nach Anspruch 2, wobei die optischen Elemente dazu konfiguriert sind, sich in seitliche und entgegengesetzte Richtungen zu verschieben, sodass sich beide optischen Elemente in axialer Richtung bewegen, wobei die gegenseitige Verschiebung den Intralinsenraum zwischen den optischen Elementen vergrößert.

4. Akkommodierende Intraokularlinsenkonstruktion nach einem der vorstehenden Ansprüche, wobei eine Kombination aus mindestens zwei mindestens teilweise kubisch ausgebildeten oder frei geformten Oberflächen dazu konfiguriert ist, eine Linse mit variabler optischer Leistung bereitzustellen, deren Grad der optischen Leistung von dem Grad der einander entgegengesetzten Bewegungen der optischen Elemente in einer seitlichen Richtung abhängt.

5. Akkommodierende Intraokularlinsenkonstruktion nach einem der vorstehenden Ansprüche 1 bis 4, wobei eine Kombination aus mindestens zwei mindestens teilweise sphärisch optisch ausgebildeten Oberflächen dazu konfiguriert ist, eine Linse mit variabler optischer Leistung bereitzustellen, deren Grad der optischen Leistung von dem Grad der Bewegungen der Elemente in einer axialen Richtung abhängt.

6. Akkommodierende Intraokularlinsenkonstruktion nach Anspruch 5, wobei die axialen Bewegungen der optischen Elemente Bewegungen in entgegengesetzte Richtungen entlang der optischen Achse sind.

7. Akkommodierende Intraokularlinsenkonstruktion nach Anspruch 5, wobei die axialen Bewegungen der optischen Elemente in derselben Richtung entlang der optischen Achse erfolgen.

8. Akkommodierende Intraokularlinsenkonstruktion nach einem der vorstehenden Ansprüche, wobei die mindestens zwei optischen Elemente eine sphärische optische Form einer positiven optischen Leistung umfassen und wobei die Linsenkonstruktion mit einer mechanischen Konstruktion bereitgestellt ist, die dazu konfiguriert ist, mindestens zwei optische Elemente in derselben Richtung entlang der optischen Achse zu bewegen.

9. Akkommodierende Intraokularlinsenkonstruktion nach einem der vorstehenden Ansprüche wobei mindestens ein optisches Element eine sphärische optische Form einer positiven optischen Leistung umfasst und wobei mindestens ein weiteres optisches Element eine sphärische optische Form einer negativen optischen Leistung umfasst, wobei die Linsenkonstruktion angepasst ist, um beide Elemente in entgegengesetzte Richtungen entlang der optischen Achse zu bewegen.

10. Akkommodierende Intraokularlinsenkonstruktion nach einem der vorstehenden Ansprüche, wobei die optischen Elemente außerdem mindestens zwei zusätzliche frei geformte optische Oberflächen umfassen, die dazu konfiguriert sind, eine variable optische Leistung von mindestens einer zusätzlichen Aberration bereitzustellen, deren Grad der optischen Leistung von dem Grad der einander entgegengesetzten Bewegungen der Elemente in einer seitlichen Richtung abhängt.

11. Akkommodierende Intraokularlinsenkonstruktion nach Anspruch 10, wobei die zusätzlichen frei geformten Oberflächen dazu konfiguriert sind, eine variable Korrektur von variablem Astigmatismus bereitzustellen.

12. Akkommodierende Intraokularlinsenkonstruktion nach Anspruch 10 oder 11, wobei die zusätzlichen frei geformten Oberflächen dazu konfiguriert sind, eine variable Korrektur von variablem Koma bereitzustellen.

13. Akkommodierende Intraokularlinsenkonstruktion nach einem der vorstehenden Ansprüche, wobei die optischen Unterelemente innerhalb eines optischen Elements (in der Linsenkonstruktion) in aneinanderstoßendem Kontakt stehen und dazu konfiguriert sind, eine Kombination aus lateralen und axialen Bewegungen der optischen Elemente und/oder der optischen Unterelemente bereitzustellen.

14. Akkommodierende Intraokularlinsenkonstruktion nach einem der vorstehenden Ansprüche, wobei die mindestens zwei unabhängigen Scharniersätze einen Scharniersatz umfassen, der angepasst ist, um laterale Bewegung der optischen Elemente bereitzustellen, und einen Scharniersatz, der angepasst ist, um axiale Bewegung der optischen Elemente bereitzustellen.

15. Akkommodierende Intraokularlinsenkonstruktion nach einem der vorstehenden Ansprüche, wobei mindestens eine optische Oberfläche wie eine kubische Formfunktion, insbesondere wie eine hermitische Formfunktion, geformt ist.

## Revendications

1. Construction de lentille intraoculaire d'accommodation comprenant une lentille et un axe optique, dans laquelle la lentille comprend au moins deux éléments optiques, chaque élément comprenant deux surfaces optiques,
dans laquelle les éléments optiques sont mutuellement couplés élastiquement par au moins une haptique élastique, et
dans laquelle au moins un élément optique, de préférence chaque élément optique, comprend au moins deux sous-éléments optiques au moins un sous-élément optique comprenant une surface optique qui est au moins partiellement formée selon une fonction cubique ou de forme libre et dans laquelle au moins un autre sous-élément optique comprend au moins une surface optique qui est au moins partiellement de forme sphérique, et
dans laquelle l'haptique élastique est configurée pour fournir, lors de la compression de la construction de lentille dans une direction latérale, ladite direction latérale étant dirigée perpendiculairement à l'axe optique, une combinaison de mouvements latéraux dans la direction latérale et de mouvements axiaux dans la direction axiale des éléments optiques parmi les éléments optiques,
dans laquelle les mouvements axiaux sont dirigés le long de l'axe optique, et dans laquelle la construction de lentille comprend une combinaison d'au moins deux ensembles indépendants de charnières, en particulier deux ensembles indépendants de charnières, comprenant au moins une charnière adaptée pour fournir un mouvement latéral indépendant des éléments optiques et au moins une charnière adaptée pour fournir un mouvement axial indépendant des éléments optiques.

2. Construction de lentille intraoculaire d'accommodation selon la revendication 1, comprenant deux éléments optiques, dans laquelle les deux éléments optiques sont placés sur des côtés opposés de la construction de lentille et sont divisés par un espace intralenticulaire entre les éléments optiques.

3. Construction de lentille intraoculaire d'accommodation selon la revendication 2, dans laquelle les éléments optiques sont configurés pour se décaler dans des directions latérales et opposées, de sorte que les deux éléments optiques se déplacent dans la direction axiale, lequel déplacement mutuel augmente l'espace intralenticulaire entre les éléments optiques.

4. Construction de lentille intraoculaire d'accommodation selon l'une quelconque des revendications précédentes, dans laquelle une combinaison d'au moins deux surfaces au moins partiellement formées selon une fonction cubique ou de forme libre est configurée pour fournir une lentille à puissance optique variable dont le degré de puissance optique dépend du degré des mouvements mutuellement opposés des éléments optiques dans une direction latérale.

5. Construction de lentille intraoculaire d'accommodation selon l'une quelconque des revendications précédentes 1 à 4, dans laquelle une combinaison d'au moins deux surfaces de forme optique au moins partiellement sphérique est configurée pour fournir une lentille de puissance optique variable dont le degré de puissance optique dépend du degré des mouvements des éléments dans une direction axiale.

6. Construction de lentille intraoculaire d'accommodation selon la revendication 5 dans laquelle les mouvements axiaux des éléments optiques sont des mouvements dans des directions opposées le long de l'axe optique.

7. Construction de lentille intraoculaire d'accommodation selon la revendication 5 dans laquelle les mouvements axiaux des éléments optiques sont dans la même direction le long de l'axe optique.

8. Construction de lentille intraoculaire d'accommodation selon l'une quelconque des revendications précédentes, dans laquelle au moins deux éléments optiques comprennent une forme optique sphérique d'une puissance optique positive et dans laquelle la construction de lentille est dotée d'une construction mécanique configurée pour déplacer au moins les deux éléments optiques dans la même direction le long de l'axe optique.

9. Construction de lentille intraoculaire d'accommodation selon l'une quelconque des revendications précédentes, dans laquelle au moins un élément optique comprend une forme optique sphérique d'une puissance optique positive et au moins un autre élément optique comprend une forme optique sphérique d'une puissance optique négative, la construction de la lentille étant adaptée pour déplacer les deux éléments dans des directions opposées le long de l'axe optique.

10. Construction de lentille intraoculaire d'accommodation selon l'une quelconque des revendications précédentes, dans laquelle les éléments optiques comprennent également au moins deux surfaces optiques supplémentaires de forme libre configurées pour fournir une puissance optique variable d'au moins une aberration supplémentaire dont le degré de puissance optique dépend du degré des mouvements mutuellement opposés des éléments dans une direction latérale.

11. Construction de lentille intraoculaire d'accommodation selon la revendication 10, dans laquelle les surfaces supplémentaires de forme libre sont configurées pour fournir une correction variable d'astigmatisme variable.

12. Construction de lentille intraoculaire d'accommodation selon la revendication 10 ou **11,** dans laquelle les surfaces supplémentaires de forme libre sont configurées pour fournir une correction variable de coma variable.

13. Construction de lentille intraoculaire d'accommodation selon l'une quelconque des revendications précédentes, dans laquelle les sous-éléments optiques à l'intérieur d'un élément optique sont en contact de butée (dans la construction de lentille) et sont configurés pour fournir une combinaison de mouvements latéraux et axiaux des éléments optiques et/ou des sous-éléments optiques.

14. Construction de lentille intraoculaire d'accommodation selon l'une quelconque des revendications précédentes, dans laquelle au moins deux ensembles indépendants de charnières comprennent un ensemble de charnières adaptées pour fournir un mouvement latéral des éléments optiques et un ensemble de charnières adaptées pour fournir un mouvement axial des éléments optiques.

15. Construction de lentille intraoculaire d'accommodation selon l'une quelconque des revendications précédentes, dans laquelle au moins une surface optique est formée comme une fonction de forme cubique, en particulier comme une fonction de forme hermitienne.
